# EUROPEAN PATENT SPECIFICATION

(11) **EP 1 397 329 B1**
(45) Date of publication and mention of the grant of the patent: **07.03.2007**
(21) Application number: 02748794.1
(22) Date of filing: 18.06.2002
(51) Int. Cl.: C07C 2/32

(54) **PROCESS FOR THE PREPARATION OF OLIGOMERS**
VERFAHREN ZUR HERSTELLUNG VON OLIGOMEREN
PROCEDE DE PREPARATION D'OLIGOMERES

(30) Priority: 20.06.2001 EP 01305353
(43) Date of publication of application: 17.03.2004
(73) Proprietor: SHELL INTERNATIONALE RESEARCH MAATSCHAPPIJ B.V., 2596 HR Den Haag (NL)
(72) Inventor: DE BOER, Eric, Johannes, Maria, NL-1031 CM Amsterdam (NL); DEULING, Hendrikus, Hyacinthus, NL-1031 CM Amsterdam (NL); VON HEBEL, Klaas, Lambertus, NL-1031 CM Amsterdam (NL); RUISCH, Bart, Johan, NL-1031 CM Amsterdam (NL); VAN ZON, Arie, NL-1031 CM Amsterdam (NL)
(86) International application number: PCT/EP2002/006739
(87) International publication number: WO 2003/000628

(56) References cited:
- WO-A-99/52631

## Description

The present invention relates to a process for the preparation of oligomers from one or more olefinically unsaturated monomers comprising three or more carbon atoms using a catalyst system based on a specific titanium bisamide compound. The invention also relates to specific propylene homo-oligomers, which can be obtained by this process.

Bisamido transition metal compounds are known to be active as catalysts for polymerizing α-olefins. For instance, International patent applications Nos. WO-92/12162 and WO-96/27439 both disclose the use of such compounds in conjunction with alumoxanes or boron compounds as catalysts for polymerizing ethylene and propylene. In both patent specifications only zirconium and hafnium bisamide compounds are exemplified.

The use of bisamido zirconium complexes for oligomerizing ethene is described in WO-99/52631.

Copending European patent application No. 00203330.6 discloses the use of catalyst systems obtainable by contacting a titanium bisamide compound, one or more activating boron-containing cocatalysts and possibly one or more aluminoxanes or aluminium alkyls in the homopolymerization of ethylene and copolymerization of ethylene with higher olefins.

The present invention relates to a process for the oligomerization of one or more C₃ to C₃₀ aliphatic mono-α-olefins using the catalyst system described in the aforesaid copending European patent application No. 00203330.6.

Accordingly, the present invention relates to a process for the oligomerization of one or more C₃ to C₃₀ aliphatic mono-α-olefins, which process comprises reacting the one or more C₃ to C₃₀ aliphatic mono-α-olefins under oligomerization conditions in the presence of an effective amount of a catalyst system based on
(a) a titanium bisamide compound of general formula (I) or its dimer of general formula (II) 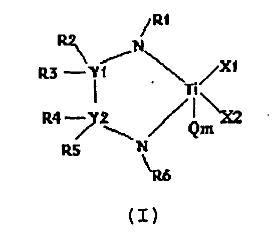 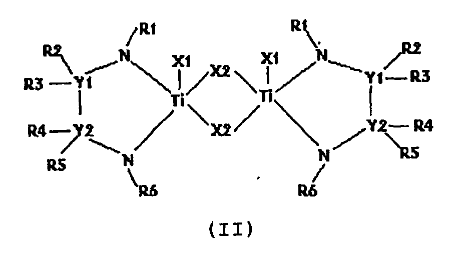 wherein:
   Y¹ and Y² independently represent silicon (Si), germanium (Ge) or tin (Sn);
   X¹ and X² independently represent a substituted or unsubstituted hydrocarbon group, in which one or more of the carbon atoms may be replaced by a Si atom, while in formula (I) X¹ and X² may also independently represent hydrogen or halogen or together form a ring structure; and
   R¹, R², R³, R⁴, R⁵ and R⁶ independently represent hydrogen, halogen or a substituted or unsubstituted hydrocarbon group;
   Q represents a neutral Lewis base; and
   m is 0 or 1;
(b) one or more activating cocatalysts selected from the group consisting of
   (i) compounds of formula U⁺Z⁻, wherein U⁺ represents a cation capable of reacting irreversibly with one or more of the substituents X¹ and X² of the compound of formula (I) or (II) and Z⁻ represents a compatible non-coordinating anion comprising at least one boron atom; and
   (ii) neutral strong Lewis acids comprising at least one boron atom; and optionally, but in any event if in the compound of formula (I) or (II) at least one of X¹ or X² represents halogen:
(c) one or more aluminium alkyls or alumoxanes.

It was surprisingly found that when using the titanium bisamide catalyst-based catalyst system defined above for polymerizing propylene a very high selectivity for vinyl group-terminated polymer chains (-CH=CH₂) was attained, whereas an extensive array of Group 4 metal-based catalyst, including similar zirconium bisamide catalysts, under identical conditions produce predominantly vinylidene-terminated polymer chains (-C(CH₃)=CH₂). Furthermore, an additional advantage of the present invention is that when C4 or higher olefins are used as monomer(s) the number of branches in the final oligomer molecule is less than when using the corresponding zirconium bisamide catalysts. Yet another major advantage is that in the present process a high proportion of linear dimer molecules is formed. Such dimer molecules are, for instance, particularly useful as comonomers in the production of linear low density polyethylene. It was surprisingly found that the titanium bisamide catalyst-based catalyst system defined above results in a higher proportion of dimers than would be expected in a normal oligomerization reaction obeying Schulz-Flory distribution, wherein the relative amounts of oligomer fractions are constant for two successive fractions and thus formation of dimers is not normally favoured over trimers, tetramers, pentamers etc.

The expressions "oligomerization", "oligomers", "oligomerized" and words derived from any of these expressions as used throughout this specification cover every reaction between two or more olefinically unsaturated monomer molecules, either the same or different, as well as any molecule resulting from such reaction up to a molecular weight of 10,000. Accordingly, the range of molecular weights covered ranges from 84 (i.e. two propylene molecules) to 10,000. The expression "molecular weight" as used throughout this specification refers to the molecular weight as determined by gas chromatography and NMR.

The olefinically unsaturated monomers, which can be oligomerized in accordance with the present process, are the C₃ to C₃₀ aliphatic mono-α-olefins, more preferably the C₃ to C₁₂ aliphatic mono-α-olefins. The process of the present invention comprises reacting one or more of these mono-α-olefins.

The titanium bisamide compound of formula (I) and its dimer of formula (II) can both be used as component (a) of the catalyst system. It is, however, preferred to use the compound of formula (I). In the titanium bisamide compound of formula (I) and its dimer of formula (II), the atoms Y¹ and Y² are preferably identical and represent Si.

The groups X¹ and X² are preferably selected from hydrogen, halogen, C₁-C₅ alkyl, 4-alkylphenyl, phenyl and benzyl. Examples of very suitably C₁-C₅ alkyl groups are methyl, ethyl, propyl and n-butyl. The groups X¹ and X² can also form a ring having from 4 to 8 members together with the titanium atom. Most preferably, X¹ and X² are the same and selected from halogen, methyl and benzyl groups.

The groups R¹, R², R³, R⁴, R⁵ and R⁶ are the same or different and are preferably selected from hydrogen, C₁-C₅ alkyl, trimethylsilyl, C₅-C₈ cycloalkyl, phenyl and benzyl. Suitable C₁-C₅ alkyl groups in this connection are methyl, ethyl, propyl, isopropyl, n-butyl, isobutyl, tert-butyl and tert-amyl, while from the C₅-C₈ cycloalkyl groups cyclopentyl and cyclohexyl are preferred. In addition to the preferred options mentioned above, groups R¹, R², R³, R⁴, R⁵ and R⁶ may also represent other groups like dimethylphenyl, diisopropylphenyl, tri-tert-butylphenyl, diphenylethyl and triphenylmethyl. It was found that the best results are obtained when R¹ and R⁶ are groups, which provide steric hindrance to the Ti atom. Thus, bulky groups like tert-butyl and 2-phenyl-isopropyl are very suitable groups. In a very much preferred embodiment R², R³, R⁴ and R⁵ are methyl groups and R¹ and R⁶ are tert-butyl groups.

Q is a neutral Lewis base, which may (m=1) or may not (m=0) be present. Examples of suitable Lewis bases are diethylether, tetrahydrofuran, diethylaniline and dimethylaniline.

Preferred compounds of formula (I) and (II) are those wherein the groups Y¹, Y², X¹, X², R¹, R², R³, R⁴, R⁵ and R⁶ have the preferred meanings as indicated hereinbefore and m=0. The most preferred compounds of formula (I) are {1,2-bis(t-butylamide)-tetramethyldisilane} titanium dibenzyl {Me₂SiN-*t*-Bu)₂} Ti(CH₂Ph)₂ and {1,2-bis(t-butylamide)-tetramethyldisilane} titanium dichloride {Me₂SiN-*t*-Bu)₂} TiCl₂.

The bisamide compounds of formula (I) and (II) can be prepared by methods known in the art, more specifically as described in WO-96/27439 and Chem.Ber./Recl. (1997), 130(3), 399-403.

Component (b) of the catalyst system used in the process of the present invention is at least one activating cocatalyst selected from neutral strong Lewis acids comprising at least one boron atom and compounds of formula U⁺Z⁻, wherein U⁺ represents a cation capable of reacting irreversibly with one or more of the substituents X¹ and X² of the compound of formula (I) or (II) and Z⁻ represents a compatible non-coordinating anion-comprising at least one boron atom, suitably a boron-containing bulky and labile anion which is non-coordinating under the reaction conditions applied. Suitable strong Lewis acids are those Lewis acids capable of extracting at least one of the radicals X¹ and X² from the compound of formula (I) or (II), thereby also contributing an anion Z⁻. The anion Z⁻ must be capable of stabilising the active catalyst species resulting from the reaction between the compound of formula (I) or (II) with the activating cocatalyst and must be sufficiently labile in order to be replaceable by an olefinic substrate during the oligomerization reaction. Compounds of formula U⁺Z⁻ are preferred cocatalysts.

In a preferred embodiment the cocatalyst is a compound of formula U⁺Z⁻, wherein U⁺ represents a quaternary ammonium cation, preferably dimethylanilinium [PhMe₂NH]⁺ or tri(n-butyl)ammonium [Bu₃NH]⁺. U⁺ may also represent a non proton-donating cation, in particular a metal cation like a silver ion or a triphenylcarbonium ion. Z⁻ suitably represents a borate anion of formula [B(R⁸)₄]⁻, wherein the groups R⁸ independently represent hydrogen or halogen-substituted (most suitably fluorine-substituted) or unsubstituted C₁-C₁₀ alkyl, C₅-C₈ cycloalkyl, phenyl, C₇-C₁₅ alkylaryl or C₇-C₁₅ arylalkyl groups. Specific examples include tetraphenyl borate [B(C₆H₅)₄]⁻, tetrakis (pentafluorophenyl) borate [B(C₆F₅)₄]⁻, tetrakis (3,5-bis-trifluoromethyl-phenyl) borate [B(3,5-(CF₃)₂-C₆H₃)₄]⁻ and tetrakis (4-fluorophenyl) borate [B(4-F-C₆H₄)₄]⁻, of which tetrakis (pentafluorophenyl) borate is most preferred. Furthermore, Z⁻ may contain multiple boron atoms, such as in carborates like 1-carbodecarborate [B₁₁CH₁₂]⁻. The preferred compounds of formula U⁺Z⁻ are those in which U⁺ and Z⁻ have a preferred meaning as indicated hereinbefore. A very much preferred compound of formula U⁺Z⁻ is dimethylanilinium tetrakis (pentafluorophenyl) borate [PhMe₂NH]⁺ [B(C₆F₅)₄]⁻.

The boron-containing component (b) can be prepared by methods known in the art, such as e.g. disclosed in WO-96/27439.

The catalyst system may in addition be based on one or more aluminium alkyls or alumoxanes (component (c)). If in the compound of formula (I) or (II) at least one of X¹ and X² represents halogen, then this component (c) is anyhow present; in that case it acts as alkylating agent. In general, however, the presence of on one or more aluminium alkyls or alumoxanes is advantageous, as they act as scavengers for removing oligomerization poisons, such as molecules containing N-, O-, S- or P-donor atoms.

Suitable aluminium alkyls are, for example, compounds of the general formulas HₓAlR⁹₃₋ₓ or H_{y}Al₂R⁹_{6-y'} wherein x and y are (possibly non-integer) numbers ranging from 0 to 1 and the groups R⁹ independently represent halogen, C₁-C₂₀ alkyl or alkenyl, C₃-C₂₀ cycloalkyl, C₆-C₂₀ aryl, C₇-C₂₀ alkylaryl and C₇-C₂₀ arylalkyl groups with branched C₁-C₂₀ alkyl and C₇-C₂₀ alkylaryl groups being preferred. Mixtures of different aluminium alkyls can also be used. Particularly preferred aluminium alkyl compounds are trimethyl aluminium, tris(2,4,4-trimethylpentyl) aluminium, tri-isobutyl aluminium, tris(2,3,3-trimethyl-butyl) aluminium, tris(2,3-dimethyl-butyl) aluminium, tris(2-phenyl-propyl) alumium, tris[2-(4-fluorophenyl)propyl] aluminium and tris[2-(4-chlorophenyl)-propyl] aluminium.

Suitable alumoxanes that can be used as component (c) may be linear alumoxanes of general formula (III) or cyclic alumoxanes of formula (IV) 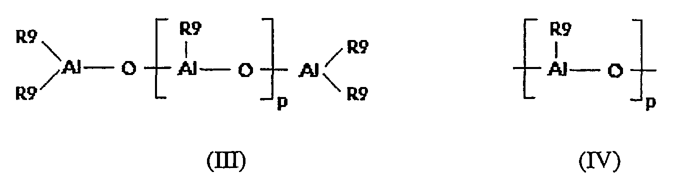 wherein R⁹ has the meaning as described hereinbefore and p is an integer ranging from 3 to 40. These alumoxanes may be obtained by methods known in the art by reacting water with an aluminium alkyl of general formula HₓAlR⁹₃₋ₓ or H_{y}Al₂R⁹_{6-y} with the proviso R⁹ is not halogen. In this case the molar ratio of Al to water may suitably range from 1:1 to 100:1. Suitable organometallic aluminium compounds are also those represented by formula (II) in EP-A-0 575 875 and those represented by formula (II) in WO-96/02580.

The amount in which the catalyst components are used may vary within broad limits. Typically, the molar ratio between components (a) and (b) of the catalyst system is in the range of from 0.1:1 to 5:1, preferably 0.2:1 to 2.5:1 and more preferably 0.3:1 to 1.1:1. Component (c), if present, is suitably used in such amount that the molar ratio between the aluminium in component (c) and the Ti in component (a) ranges from 10:1 to 10,000:1, preferably 20:1 to 5000:1 and more preferably 20:1 to 1000:1.

The catalyst system may be used as a homogeneous catalyst by adding the catalyst components to the liquid reaction medium, so that the active catalyst will be formed in situ. Alternatively, the catalyst system may be supported on a solid inert support material, thereby also enabling the oligomerization reaction to take place in the gas phase. If used on an inert support material, the catalyst components are deposited on the support material, e.g. by impregnation with one or more suitable impregnating solutions. Suitable support materials include refractory oxides such as silica, silica-alumina, alumina, titania, zirconia and magnesia, as well as zeolitic carriers. Furthermore, olefin polymers or prepolymers may be used as support materials. Examples include polyethylenes, polypropylenes and styrene/divinylbenzene copolymers.

The process of the present invention can be carried out in the liquid phase or in the gas phase. If carried out in the liquid phase the reactant monomers and oligomer formed may provide the reaction medium, but alternatively an inert hydrocarbon solvent may be used. Suitable solvents include aromatic hydrocarbons like toluene or aliphatic hydrocarbons like pentane, hexane, heptane, isobutane or cyclohexane.

The conditions under which the oligomerization of the present invention is carried out are not particularly critical and may vary within broad limits. Typically, however, reaction temperatures will be in the range of from -100 °C to +200 °C, preferably 0 °C to 100 °C and more preferably 10 °C to 90 °C. The oligomerization pressure is typically in the range of from 0.5 to 100 bara, preferably 1 to 50 bara.

The process of the present invention is suitable for preparing homo-oligomers of propylene and higher mono-α-olefins, up to C₃₀ olefins, and co-oligomers of such olefins with each other or with other olefinically unsaturated monomers mentioned hereinbefore. The length of the oligomer chain prepared may start from two monomer molecules and suitably does not exceed twenty, more suitably ten and most suitably five monomer molecules. Nevertheless, longer chains may be prepared, but the molecular weight will anyhow not exceed 10,000.

Preferred oligomers to be prepared in accordance with the present invention are homo-oligomers of C₃ to C₃₀ aliphatic mono-α-olefins, more preferably C₃ to C₁₂ aliphatic mono-α-olefins, having a molecular weight up to 10,000, preferably up to 5000 and more preferably up to 1000. Particularly preferred oligomers are homo-oligomers of propylene, 1-butene, 1-pentene, 1-hexene, 1-decene and 1-dodecene. However, co-oligomers of propylene with other C₄ to C₁₂ mono-α-olefins can also be very suitably prepared in the process according to the present invention.

It was surprisingly found that when using the titanium bisamide catalyst system of the present invention and propylene as the sole monomer, a very high proportion of the oligomer chains formed is terminated with a vinyl group (i.e. -CH=CH₂). By means of comparison: structurally corresponding zirconium bisamide catalyst systems as disclosed in WO-96/27439 result in a much higher proportion of vinylidene-terminated propylene oligomer chains (i.e. - (CH₂)C=CH₂) The advantage of having a vinyl end-group is that the oligomer can be used as comonomer in other polymerization reactions, such as in the preparation of linear low density polyethylene. This is not possible with oligomer molecules having a vinylidene end group.

As indicated hereinbefore, further advantages of the present process are that a very high proportion of linear dimers is formed instead of dimers containing a branch. If propylene is used as the monomer a substantial part of these dimers will have a vinyl end group. If a C4 or higher mono-α-olefins is used as monomer, the linear dimer formed will have an internal olefinic bond. This surprising finding is thought to be the result of the very specific termination mechanism caused by the titanium bisamide catalyst used.

Accordingly, in a further aspect the present invention relates to a mixture of homo-oligomers of propylene obtainable by the process of the present invention, which mixture comprises oligomers of propylene based on up to 20, preferably up to 10 and more preferably up to 5, propylene molecules, wherein at least 50 mole% of the oligomer chains have a terminal vinyl group.

The invention also relates to homo-oligomers of The invention will now be illustrated by the following examples without limiting the scope of the invention to these particular embodiments.

### Examples

### Example 1 Preparation of

### (t-BuNSiMe₂SiMe₂N-t-Bu)Ti(CH₂Ph)₂

### A. [(t-BuNSiMe₂SiMe₂N-t-Bu)²⁻][Li⁺]₂ [1]

To a solution of (*t*-BuHNSiMe₂SiMe₂NH-*t*-Bu), (2.5 g, 9.6 mMole) in 25 ml of hexane, 12.6 ml of 1.6 M *n*-BuLi/hexane solution (20.2 mMole) was added slowly at -78 °C. The reaction mixture was stirred for 1.5 hours while slowly warming to room temperature. The resulting off-white precipitate was decanted and the washed with 10 ml of hexane. After removal of the hexane layer and drying the solids, 2.5 g of product was isolated.
¹H-NMR (thf-*d₈*) : δ 1.11, (s, 18H, *CMe₃),* 0.04 (s, 12H, Si*Me₂*).

### B. (t-BuNSiMe₂SiMe₂N-t-Bu)TiCl₂ [2]

To a stirred solution of TiCl₄ (5.3 g, 5.5 mMole) in 25 ml of toluene was added slowly a suspension of **[1]** (1.5 g, 5.5 mMole) at -78 °C. The reaction mixture is allowed to warm to room temperature slowly and stirred for another 4 hours. Subsequently the volatiles were evaporated and the dark residue extracted twice with 15 ml of hexane. The combined hexane layers were cooled to -40 °C affording a crystalline product. After removing the mother liquor by syringe, the remaining crystals were washed with 5 ml of diethyl ether, dried under vacuum, and subsequently isolated. Yield 0.14 g of **[2]**.
¹H-NMR (C₆D₆) : δ 1.44 (s,18H,C*Me*₃), 0.08 (s,12H,Si*Me*₂)

### C. (t-BuNSiMe₂SiMe₂N-t-Bu)Ti(CH₂Ph)₂ [3]

To a stirred suspension of **[2]** (0.1 g, 0.26 mMole) in 5 ml of diethyl ether were added 5 ml of a 1M solution of PhCH₂MgCl (0.53 mMole) in diethyl ether at -78 °C. After warming the solution to room temperature, the reaction mixture was stirred for another 2 hours and subsequently all volatiles were evaporated off under vacuum. The remaining residue was extracted with 1.5 ml of hexane and the resulting hexane solution cooled overnight to -40 °C. The resulting crystalline material was isolated after drying under vacuum. Yield 25 mg of **[3]**.
¹H-NMR (C₆D₆) : δ 7.08 (t, 4H, *m*-Bz), 6.94 (t, 4H, *o*-Bz), 6.92 (t, 2H, *p*-Bz), 2.89 (s, 4H, Ti*CH*₂) 1.52 (s, 18H, C*Me₃*), 0.08 (s, 12H, Si*M*e*₂*).

### Example 2

Propene oligomerization in solution was carried out in a 1-litre steel autoclave equipped with jacket cooling with a heating/cooling bath and a turbine/gas stirrer. In order to remove traces of oxygen from the reactor, it was evacuated overnight at <0.1 mbara, at 70 °C. The temperature was then decreased to 50 °C and the autoclave was pressurized with nitrogen (4-5 bara). Subsequently, the reactor was scavenged with a solution of tetra-iso-butyl alumoxane (TIBAO) (300 mg) in *iso*-octane (140 g) and stirring was applied for 30 minutes. The reactor contents were discharged via a tap in the base of the autoclave. The reactor was evacuated to 4 mbara and loaded with 243 g of iso-octane as solvent and 2 g of *n*-hexylbenzene as an internal GC standard; the reactor was heated to 70 °C, pressurized with propylene and equilibrated for 15 minutes. Subsequently, [(*t*-BuNSiMe₂SiMe₂N-*t*-Bu) TiCH₂Ph⁺] [B(C₆F₅)₄⁻] was prepared *in situ* by reaction of **[3]** (0.026 mMole) with 0.026 mMole [PhNMe₂H⁺] [B(C₆F₅)₄⁻] (DANFABA) in toluene and added to the reactor using a catalyst injection device. The reaction was allowed to proceed for 1 hour after which the reaction was terminated by venting rapidly excess propylene, removal of the reactor contents via the base tap, and exposing the obtained liquid product to air. The reaction products obtained were characterized by means of GC and NMR.

From the GC and NMR analyses followed that 125 g of propene oligomers were formed. For the C₆-fraction the following olefins have been identified by GC: Hexene-1 (56% by weight), *cis-*hexene-2 (24%), *trans*-hexene-2 (8%), 4-methylpentene-1 (8%), *cis*-4-methylpentene-2 (3%), remainders (1%). The C₉-fraction contained 5-methyloctene-1 (52%). The oligomers did not contain vinylidene-terminated (R-C(CH₃)=CH₂) olefins in more than trace amounts.

### Example 3

Propene oligomerization in liquid propylene at 50°C was carried out in a 5-litre autoclave, dried and scavenged prior to use, containing 1600 g of liquid propene and 3.0 mMole TIBAO. Catalyst used was made *in situ* by stirring 11.4 µMole of [3] with an equimolar amount of DANFABA for 20 minutes in 20 ml of toluene containing 0.1-0.2 mMole of TIBAO. Using a injection system, the catalyst was introduced in the reactor. The reaction was stopped after 60 minutes and the liquid contents of the reactor analysed.

The yield of the reaction was 352 g of propene oligomers of which the C₆ fraction consisted of hexene-1 (78%), *cis*-hexene-2 (10%), trans-hexene-2 (3%), 4-methylpentene-1 (7%), *cis*-4-methylpentene-2 (1%), remainders (<1%). The C₉-fraction contained 5-methyloctene-1 (75%). The average molecular weight of the product was 224. The oligomers did not contain vinylidene-terminated (R-C(CH₃)=CH₂) olefins in more than trace amounts.

### Example 4

Propene oligomerization in liquid propylene at 70°C was carried out in a 5-litre autoclave, dried and scavenged prior to use, containing 1600 g of liquid propene and 3.0 mMole TIBAO. Catalyst used was made *in situ* by stirring 20.5 µMole of [3] with an equimolar amount of DANFABA for 20 minutes in 20 ml of toluene containing 0.1-0.2 mMole of TIBAO. Using a injection system, the catalyst was introduced in the reactor. The reaction was stopped after 45 minutes and the liquid contents of the reactor analysed.

The yield of the reaction was 260 g of propene oligomers of which the C₆ fraction consisted of hexene-1 (76%), *cis*-hexene-2 (11%), *trans*-hexene-2 (4%), 4-methylpentene-1 (8%), *cis*-4-methylpentene-2 (1%), remainders (<1%). The C₉-fraction contained 5-methyloctene-1 (75%). The average molecular weight of the product was 266. The oligomers did not contain vinylidene-type (R-C(CH₃)=CH₂) olefins in more than trace amounts.

### Comparative example 1

[(*t*-BuNSiMe₂SiMe₂N-*t*-Bu) ZrCH₂Ph⁺] [B (C₆F₅)₄⁻], **[3-Zr],** was prepared according to a procedure similar to that described in WO-96/27439.

**[3-Zr]** was reacted with [Ph₃C⁺] [B(C₆F₅)4⁻] and excess propene in bromobenzene at 25°C for 3 hours in a small glass reactor at 1 bar. NMR analysis of the products showed the formation of a-tactic propene oligomers with the characteristic unsaturated -(CH₃)CH=CH₂ end-group.

### Comparative example 2

To a 25 ml autoclave dried and evacuated prior to use, (*t*-BuNSiMe₂SiMe₂N-*t*-Bu)Zr(CH₃)₂ (0.03 mMole) which was reacted with an equimolar amount of B(C₆F₅)₃ dissolved in 10 ml of toluene, was added. Subsequently the autoclave was charged with 6.5 bar of propene and the reaction allowed to progress for 1 hour.

Analysis of the reactor contents showed the formation of hexenes (1 isomer), nonene (1 isomer), and higher propene oligomers. The hexene isomer was 2-methylpentene-1, i.e. the propene dimer with the characteristic unsaturated -(CH₃)CH=CH₂ end-group.

### Example 5

To a solution of pentene-1 (25 ml, dried over molsieves) in *iso-*octane (60 ml) containing 160 mg of scavenger TIBAO, a solution containing a mixture of 29 mg of [3], 49 mg of DANFABA, and 90 mg of TIBAO in 5 ml of toluene was added at room temperature in a glovebox. The reaction was stopped after 90 minutes by filtration of the mixture over Al₂O₃ to kill and remove the catalyst and its decomposition products. The resulting mixture was analysed and characterized by NMR and GC and found to contain pentene-1 oligomers with an average degree of polymerization between 5 and 6. The dimer fraction was shown to be a mixture of approximately equal amounts of *cis* and *trans* decene-4 and decene-3, together being 95% of the total C₁₀ fraction. Similarly, the trimer fraction predominantly contained four internal olefins having a single branched alkyl chain. The composition of the higher oligomer fractions was more difficult to determine in detail, but all these fractions have in common that olefins different from internal R₁-CH=CH-R₂ olefins are present in not more than trace amounts. Assuming response factors linearly correlating with the number of carbon atoms, the dimer over trimer and trimer over tetramer ratios in the final reaction product were calculated to be 0.96 and 0.26, respectively.

## Claims

1. Process for the oligomerization of one or more C₃ to C₃₀ alipathic mono-α-olefins, which process comprises reacting the one or more C₃ to C₃₀ aliphatic mono-α-olefins under oligomerization conditions in the presence of an effective amount of a catalyst system based on
(a) a titanium bisamide compound of general formula (I) or its dimer of general formula (II) 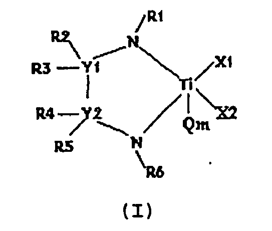 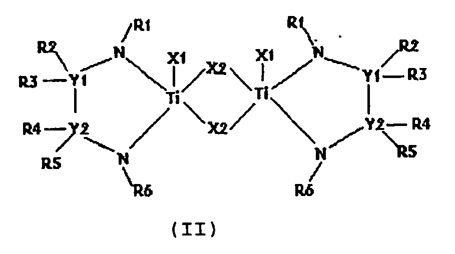 wherein:
Y¹ and Y² independently represent Si, Ge or Sn;
X¹ and X² independently represent a substituted or unsubstituted hydrocarbon group, in which one or more of the carbon atoms may be replaced by a Si atom, while in formula (I) X¹ and X² may also independently represent hydrogen or halogen or together form a ring structure; and
R¹, R², R³, R⁴, R⁵ and R⁶ independently represent hydrogen, halogen or a substituted or unsubstituted hydrocarbon group;
Q represents a neutral Lewis base; and
m is 0 or 1;
(b) one or more activating cocatalysts selected from the group consisting of
(i) compounds of formula U⁺Z⁻, wherein U⁺ represents a cation capable of reacting irreversibly with one or more of the substituents X¹ and X² of the compound of formula (I) or (II) and Z⁻ represents a compatible non-coordinating anion comprising at least one boron atom; and
(ii) neutral strong Lewis acids comprising at least one boron atom; and optionally, but in any event if in the compound of formula (I) or (II) at least one of X¹ and X² represents halogen:
(c) one or more aluminium alkyls or alumoxanes.

2. Process as claimed in claim 1, wherein the titanium bisamide compound is a compound of formula (I).

3. Process as claimed in claim 1 or claim 2, wherein Y¹ and Y² represent Si.

4. Process as claimed in any one of claims 1 to 3, wherein x¹ and X² are selected from hydrogen, halogen, C₁-C₅ alkyl, phenyl, 4-alkylphenyl and benzyl.

5. Process as claimed in any one of claims 1 to 4, wherein R¹, R², R³, R⁴, R⁵ and R⁶ are the same or different and are selected from hydrogen, C₁-C₅ alkyl, trimethylsilyl, C₅-C₈ cycloalkyl, phenyl and benzyl.

6. Process as claimed in any one of claims 1 to 5, wherein the cocatalyst is a compound of formula U⁺Z⁻, wherein U⁺ represents a quaternary ammonium cation and Z- represents a borate anion of formula [B(R⁸)₄]⁻, wherein the groups R⁸ independently represent hydrogen or halogen-substituted or unsubstituted C₁-C₁₀ alkyl, C₅-C₈ cycloalkyl, phenyl, C₇-C₁₅ alkylaryl or C₇-C₁₅ arylalkyl groups.

7. Process as claimed in claim 6, wherein U⁺ is dimethylanilinium or tri(n-butyl) ammonium and Z- is tetrakis (pentafluorophenyl) borate.

8. Process as claimed in any one of claims 1 to 7, wherein the catalyst system is based on components (a) and (b) in a molar ratio in the range of from 0.1:1 to 5:1 and component (c), if present, is used in such amount that the molar ratio between the aluminium in component (c) and the Ti in component (a) ranges from 10:1 to 10,000:1.

## Patentansprüche

1. Verfahren zur Oligomerisierung von einem oder mehreren aliphatischen C₃-C₃₀-Mono-α-olefinen, welches Verfahren das Umsetzen des einen oder der mehreren aliphatischen C₃-C₃₀-Mono-α-olefine unter Oligomerisierungsbedingungen in Gegenwart einer effektiven Menge eines Katalysatorsystems umfaßt, welches Katalysatorsystem auf
(a) einer Titanbisamidverbindung der allgemeinen Formel (I) oder deren Dimer der allgemeinen Formel (II) 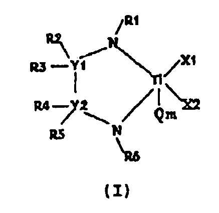 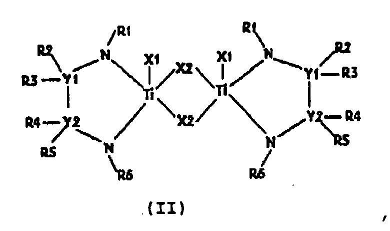 worin:
Y¹ und Y² unabhängig Si, Ge oder Sn darstellen;
X¹ und X² unabhängig eine substituierte oder unsubstituierte Kohlenstoffgruppe darstellen, worin eines oder mehrere der Kohlenstoffatome durch ein Si-Atom ersetzt sein kann, wobei in Formel (I) X¹ und X² auch unabhängig Wasserstoff oder Halogen darstellen können oder gemeinsam eine Ringstruktur ausbilden können; und
R¹, R², R³, R⁴, R⁵ und R⁶ unabhängig Wasserstoff, Halogen oder eine substituierte oder unsubstituierte Kohlenstoffgruppe darstellen;
Q eine neutrale Lewis-Base bedeutet; und
m für 0 oder 1 steht;
(b) einem oder mehreren aktivierenden Co-Katalysatoren, ausgewählt von der Gruppe bestehend aus
(i) Verbindungen der Formel U⁺Z⁻, worin U⁺ ein Kation darstellt, welches fähig ist, irreversibel mit einem oder mehreren der Substituenten X¹ und X² der Verbindung der Formel (I) oder (II) zu reagieren, und Z- ein verträgliches nichtkoordinierendes Anion darstellt, welches wenigstens ein Boratom umfaßt; und
(ii) neutralen starken Lewis-Säuren, welche wenigstens ein Boratom umfassen; und wahlweise, aber in jedem Fall, wenn in der Verbindung der Formel (I) oder (II) wenigstens einer der Reste X¹ und X² Wasserstoff darstellt:
(c) einem oder mehreren Aluminiumalkylen oder Alumoxanen, basiert.

2. Verfahren nach Anspruch 1, worin die Titanbisamidverbindung eine Verbindung der Formel (I) ist.

3. Verfahren nach Anspruch 1 oder 2, worin Y¹ und Y² Si darstellen.

4. Verfahren nach einem der Ansprüche 1 bis 3, worin X¹ und X² unter Wasserstoff, Halogen, C₁-C₅-Alkyl, Phenyl, 4-Alkylphenyl und Benzyl ausgewählt sind.

5. Verfahren nach einem der Ansprüche 1 bis 4, worin R¹, R², R³, R⁴, R⁵ und R⁶ gleich oder verschieden sind und unter Wasserstoff, C₁-C₅-Alkyl, Trimethylsilyl, C₅-C₈-cycloalkyl, Phenyl und Benzyl ausgewählt sind.

6. Verfahren nach einem der Ansprüche 1 bis 5, worin der CoKatalysator eine Verbindung der Formel U⁺Z⁻ ist, worin U⁺ ein quaternäres Ammoniumkation darstellt und Z⁻ ein Boratanion der Formel [B(R⁸)₄]⁻ darstellt, worin die Gruppen R⁸ unabhängig voneinander Wasserstoff oder Halogen-substituierte oder unsubstituierte C₁-C₁₀-Alkyl-, C₅-C₈-Cycloalkyl-, Phenyl-, C₇-C₁₅-Alkylaryl- oder C₇-C₁₅-Arylalkyl-gruppen darstellen.

7. Verfahren nach Anspruch 6, worin U⁺ ein Dimethylanilinium oder Tri(n-Butyl)ammonium ist und Z- Tetrakis(pentafluorphenyl)borat darstellt.

8. Verfahren nach einem der Ansprüche 1 bis 7, worin das Katalysatorsystem auf den Komponenten (a) und (b) in einem Molverhältnis im Bereich von 0,1:1 bis 5:1 basiert, und die Komponente (c), sofern sie vorhanden ist, in solch einer Menge verwendet wird, daß das Molverhältnis zwischen dem Aluminium in der Komponente (c) und dem Ti in der Komponente (a) von 10:1 bis 10.000:1 reicht.

## Revendications

1. Procédé d'oligomérisation d'une ou plusieurs mono-α-oléfines aliphatiques en C₃ à C₃₀, lequel procédé comprend la réaction de la mono-α-oléfine aliphatique en C₃ à C₃₀ ou plus sous des conditions d'oligomérisation en présence d'une quantité efficace d'un système de catalyseur à base :
(a) d'un composé de bisamide au titane de formule générale (I) ou de son dimère de formule générale (II) : 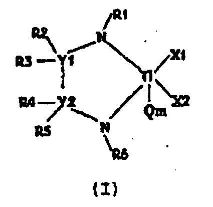 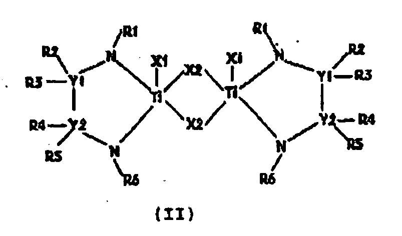 dans lesquelles :
Y¹ et Y² représentent indépendamment Si, Ge ou Sn;
X¹ et X² représentent indépendamment un groupe hydrocarboné substitué ou non substitué, dans lequel un ou plusieurs des atomes de carbone peuvent être remplacés par un atome de Si, alors que dans la formule (I) X¹ et X² peuvent également représenter indépendamment de l'hydrogène ou un halogène ou former ensemble une structure cyclique; et
R¹, R², R³, R⁴, R⁵ et R⁶ représentent indépendamment de l'hydrogène, un halogène ou un groupe hydrocarboné substitué ou non substitué;
Q représente une base de Lewis neutre; et
m est égal à 0 ou 1;
(b) d'un ou plusieurs cocatalyseurs d'activation choisis dans le groupe comprenant :
(i) les composés de formule U⁺Z⁻, dans laquelle U⁺ représente un cation pouvant réagir irréversiblement avec un ou plusieurs des substituants X¹ et X² du composé de formule (I) ou (II) et Z⁻ représente un anion non coordinant compatible comprenant au moins un atome de bore; et
(ii) les acides de Lewis forts neutres comprenant au moins un atome de bore; et éventuellement, mais en tout cas si dans le composé de formule (I) ou (II), au moins un des X¹ et X² représente un halogène :
(c) un ou plusieurs alkyles d'aluminium ou alumoxanes.

2. Procédé suivant la revendication 1, dans lequel le composé de bisamide au titane est un composé de formule (I).

3. Procédé suivant l'une ou l'autre des revendications 1 et 2, dans lequel Y¹ et Y² représentent Si.

4. Procédé suivant l'une quelconque des revendications 1 à 3, dans lequel X¹ et X² sont choisis parmi l'hydrogène, les halogènes, les groupes alkyle en C₁-C₅, phényle, 4-alkylphényle et benzyle.

5. Procédé suivant l'une quelconque des revendications 1 à 4, dans lequel R¹, R², R³, R⁴, R⁵ et R⁶ sont identiques ou différents et sont choisis parmi l'hydrogène, les groupes alkyle en C₁-C₅, triméthyl-silyle, cycloalkyle en C₅-C₈, phényle et benzyle.

6. Procédé suivant l'une quelconque des revendications 1 à 5, dans lequel le cocatalyseur est un composé de formule U⁺Z⁻, dans laquelle U⁺ représente un cation d'ammonium quaternaire et Z- représente un anion borate de formule [B(R⁸)₄]⁻, dans laquelle les groupes R⁸ représentent indépendamment de l'hydrogène ou des groupes alkyle en C₁-C₁₀, cycloalkyle en C₅-C₈, phényle, alkylaryle en C₇-C₁₅ ou arylalkyle en C₇-C₁₅ halogéno-substitués ou non substitués.

7. Procédé suivant la revendication 6, dans lequel U⁺ est du diméthylanilinium ou du tri(n-butyl) ammonium et Z⁻ est du tétrakis (pentafluorophényl) borate.

8. Procédé suivant l'une quelconque des revendications 1 à 7, dans lequel le système de catalyseur est à base de composants (a) et (b) dans un rapport molaire allant de 0,1/1 à 5/1 et le composant (c), si présent, est utilisé en une quantité telle que le rapport molaire entre l'aluminium dans le composant (c) et le Ti dans le composant (a) est de 10/1 à 10 000/1.
